# EUROPEAN PATENT SPECIFICATION

(11) **EP 1 812 449 B1**
(45) Date of publication and mention of the grant of the patent: **05.03.2008**
(21) Application number: 05810702.0
(22) Date of filing: 24.10.2005
(51) Int. Cl.: C07D 501/00

(54) **CRYSTALLINE FROM OF CEFDINIR AMMONIUM SALT AS AN INTERMEDIATED FOR THE PREPARATION OF PURE CEFDINIR**
KRISTALLINE FORM VON CEFDINIR-AMMONIUMSALZ ALS ZWISCHENPRODUKT FÜR DIE HERSTELLUNG VON REINEM CEFDINIR
SEL D'AMMONIUM CRISTALLIN DE CEFDINIR UTILISE COMME INTERMEDIAIRE POUR LA PREPARATION D'UN CEFDINIR PUR

(30) Priority: 19.11.2004 IT MI20042231
(43) Date of publication of application: 01.08.2007
(73) Proprietor: Antibioticos S.p.A., 20090 Rodano (IT)
(72) Inventor: POZZI, Giovanni, I-20045 Besana Brianza (MI) (IT); GHETTI, Paolo, I-20090 Segrate (IT); BALSAMO, Gaetano, I-20096 Pioltello (MI) (IT); ALPEGIANI, Marco, I-20132 Milano (IT); CABRI, Walter, I-20089 Rozzano (MI) (IT)
(74) Representative: Banfi, Paolo
(86) International application number: PCT/EP2005/011385
(87) International publication number: WO 2006/053625

(56) References cited:
- WO-A-98/45299
- WO-A-20/04035800
- WO-A-20/04046154
- OKAMOTO Y ET AL: "Degradation Kinetics and Isomerization of Cefdinir, a new Oral Cephalosporin, in Aqueous Solution. 1" JOURNAL OF PHARMACEUTICAL SCIENCES, AMERICAN PHARMACEUTICAL ASSOCIATION. WASHINGTON, US, vol. 85, no. 9, 1996, pages 976-983, XP002281475 ISSN: 0022-3549

## Description

### Field of the invention

The present invention relates to cephalosporins, in particular to Cefdinir and intermediates for its preparation.

### Background of the invention

Cefdinir (chemical name 7-(Z)-[2-(2-aminothiazol-4-yl)-2-hydroxyiminoacetamido]-3-vinyl-3-cephem-4-carboxylic acid) is a third generation semisynthetic cephalosporin with a wide antibacterial spectrum, particularly effective against infections caused by staphylococci and streptococci.

This antibiotic is often prepared through processes comprising the recovery of intermediates, for example salts with acids and bases, which increase the purity of the finished product without the need for further purification steps -such as chromatography- which would be troublesome or costly on an industrial scale.

WO 2004/056835 discloses a crystalline Cefdinir salt with phosphoric acid, whereas WO 02/098884 discloses crystalline Cefdinir salts with sulfuric acid and methanesulfonic acid.

US6350869 discloses a crystalline Cefdinir salt with dicyclohexylamine.

Cefdinir ammonium salt is cited in WO 2004/046154 (examples 3 and 4) as starting product for the preparation of amorphous Cefdinir monohydrate, but its recovery is not disclosed, nor is it given any indication as to its physical form.

As it is known, the cephalosporins' β-lactam ring opens in neutral and basic aqueous solutions and cephalosporins ammonium salts are in general very soluble in these solutions, therefore precipitation of cephalosporins as ammonium salts in pure and crystalline form is usually difficult.

### Disclosure of the invention

It has now been found that by adding ammonia to aqueous solutions or suspensions of Cefdinir and by properly increasing the ionic strength of the solutions or suspensions, crystalline Cefdinir ammonium salt of formula (**I**) 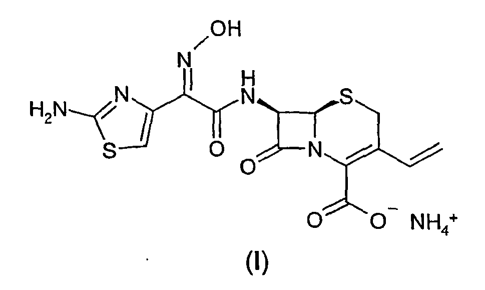 can be isolated. The salt is characterised by the following powder X-ray diffraction.

| Angle | d value | Intensity |
|---|---|---|
| (2-Theta) | (Angstrom) | (%) |
| 10.592 | 8.34491 | 39.7 |
| 12.091 | 7.31366 | 56.1 |
| 16.726 | 5.29604 | 41.2 |
| 18.023 | 4.91778 | 39.0 |
| 19.191 | 4.62106 | 96.5 |
| 19.850 | 4.46905 | 30.6 |
| 21.396 | 4.14949 | 100.0 |
| 22.876 | 3.88425 | 78.1 |
| 25.150 | 3.53798 | 49.9 |
| 25.603 | 3.47638 | 65.8 |
| 26.150 | 3.40491 | 42.2 |
| 26.845 | 3.31826 | 37.0 |
| 29.699 | 3.00563 | 37.0 |
| 30.121 | 2.96449 | 33.7 |
| 33.560 | 2.66810 | 41.0 |
| 34.658 | 2.58607 | 25.1 |
| 36.262 | 2.47524 | 18.3 |
| 36.841 | 2.43766 | 17.7 |
| 37.426 | 2.40094 | 17.6 |
| 38.220 | 2.35287 | 22.4 |
| 39.155 | 2.29881 | 16.9 |
| 40.016 | 2.25128 | 15.9 |
| 41.219 | 2.18834 | 15.6 |
| 41.779 | 2.16027 | 17.3 |
| 42.610 | 2.12004 | 16.8 |
| 46.508 | 1.95102 | 12.9 |
| 50.510 | 1.80542 | 11.9 |
| 51.487 | 1.77343 | 11.3 |
| 52.638 | 1.73733 | 13.3 |

The spectrum is also graphically reproduced in Figure 1. Cefdinir ammonium salt has an IR spectrum (in KBr) with the typical stretching of the ammonium ion at 3269 cm⁻¹, as shown in Figure 2. Moreover, the spectrum shows the stretching of the carbonyl group of the β-lactam ring at 1747 cm⁻¹ and the stretching of the amide carbonyl group at 1668 cm⁻¹. The ¹H-NMR spectrum (Figure 3) confirms the presence of the ammonium ion.

The crystalline salt of the invention shows double refraction to polarized light and has prism form, whose dimensions are up to 100-150 µm.

The crystalline salt of the invention shows high HPLC purity (higher than 99.5%) and good stability.

The salt of the invention can be obtained from a solution of Cefdinir in an aqueous solvent, obtained by adding first aqueous ammonia, so as to adjust the pH in the range of from 6 to 8, preferably from 6 to 7, and then increasing the ionic strength of the solution with an inorganic salt.

Cefdinir aqueous solutions can be obtained by dissolving Cefdinir or by working up reaction mixtures for the preparation of Cefdinir through deprotection of protected intermediates (according to literature methods).

Suitable solvents for the preparation of the salt of the invention are water or mixtures of water with alcohols, such as methanol, ethanol, n-propanol, isopropanol, n-butanol; with ketones, such as acetone, methyl-ethyl-ketone (MEK); with water-miscible ethers, such as tetrahydrofuran; with nitriles, such as acetonitrile; with esters, such as methyl acetate or ethyl acetate. Particularly preferred is the solvent mixture consisting of water and ethyl acetate.

To achieve crystallization, concentrated Cefdinir solutions, preferably with a concentration higher than 15 g/l, should be used.

If desired, after addition of ammonia, the aqueous Cefdinir solution can be treated with charcoal, then filtered or eluted through a cartridge containing charcoal, or loaded onto reverse-phase silica or adsorbing resins and then eluted.

The inorganic salts used to increase the ionic strength of the solution are selected for example from sodium chloride (NaCl), ammonium chloride (NH₄Cl), sodium monohydrogen phosphate or dihydrogen phosphate (Na₂HPO₄ and NaH₂PO₄) and ammonium dihydrogen phosphate [(NH₄)H₂PO₄].

Crystallization temperature ranges from -5°C (if this is compatible with the reaction solvent/s) to room temperature, preferably from 0°C to 10°C.

It might be advantageous to trigger the precipitation by addition of seed crystals of previously obtained salt.

The ammonium salt of the invention is recovered by filtration and washed with the same solvent mixture from which the product precipitates. If desired, the salt can be submitted to a final washing with one of the organic solvents used as cosolvents, preferably isopropanol. The product is finally dried in static or rotating oven, at 20-40°C under vacuum.

Crystalline Cefdinir ammonium salt is characterized by high HPLC purity (higher than 99.5%) and complete water solubility.

The crystalline salt of the invention can be conveniently used in a process for the preparation of Cefdinir monohydrate or crystalline form A with high purity, by dissolution of the salt in water or in water/water-miscibile solvents mixtures as described above, followed by acidification with a mineral acid, for example hydrochloric acid.

The inorganic ammonium counterion increases the solubility and dissolution rate of Cefdinir in water or water/water-miscibile solvents, and allows to prevent degradation caused by pH stress (excessive amount of base, high local pH following the base addition), which occurs in purification processes starting from Cefdinir (amorphous, crystalline form A of the Patent Fujisawa US4935507 and hydrate) or salts thereof (phosphate, sulfate, methanesulfonate and dicyclohexylamine).

The invention will be now illustrated in greater detail by means of some examples.

### Description of the figures

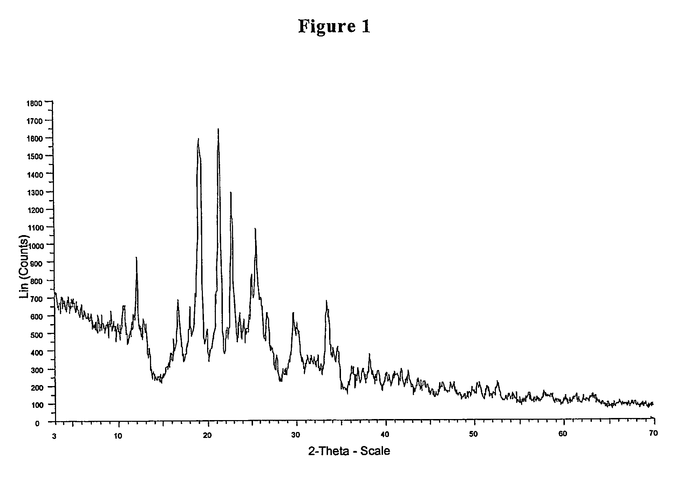

Figure 1: X ray spectrum of Cefdinir ammonium salt.

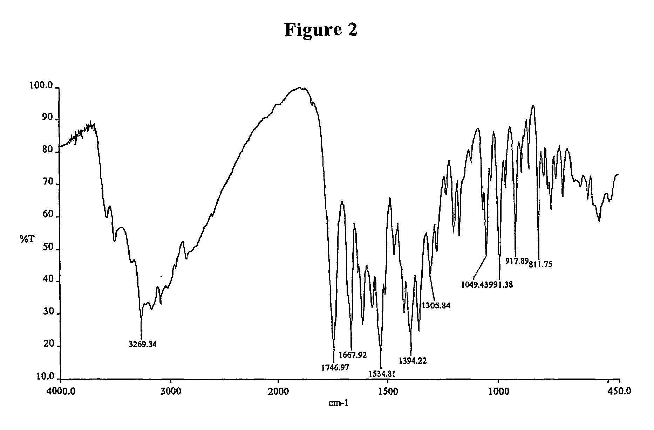

Figure 2: IR spectrum of Cefdinir ammonium salt (recorded on a Perkin Elmer Spectrum 1000 spectrometer in 1% KBr, 16 scannings, 4 cm⁻¹ resolution).

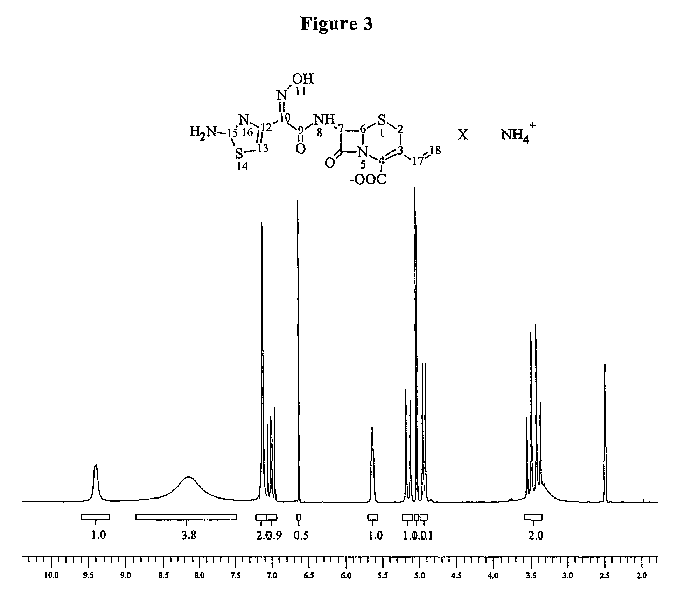

Figure 3: ¹H-NMR spectrum of Cefdinir ammonium salt recorded in DMSO-*d*₆ after 16 scannings on a 300 MHz Varian mercury spectrometer.

| **Frequency (ppm)** | **Multiplicity** | **J (Hz)** | **Integral** | **Attribution** |
|---|---|---|---|---|
| 3.50. 3.40 | AB q | 17.10 | 2H | CH₂ - 2 |
| 4.93 | d | 11.61 | 1H | CH₂ - 18 (E) |
| 5.03 | d | 4.89 | 1H | CH-6 |
| 5.14 | d | 17.72 | 1H | CH₂ - 18 (Z) |
| 5.63 | m | 4.89, 6.11 | 1H | CH-7 |
| 6.63 | s | - | 1H | CH-13 |
| 7.00 | dd | 11.61, 17.72 | 1H | CH - 17 |
| 7.12 | s | - | 2H | NH₂ |
| 8.14 | s broad | - | 4H | NH₄⁺ |
| 9.39 | d | 6.11 | 1H | NH - 8 |

### EXAMPLES

### Preparation of crystalline Cefdinir ammonium salt

### Example 1

Cefdinir phosphate (10 g) is suspended in water (112.5 ml) and ethyl acetate (7.5 ml), then a diluted ammonium hydroxide solution is added drop by drop, adjusting the pH to 6.5 and keeping the temperature at 5°C, until a solution is obtained. Seed crystals of Cefdinir ammonium salt are added and the solution is slowly stirred at 5°C for one hour. The crystallized product is filtered and washed first with water then with isopropanol. After drying crystalline Cefdinir ammonium salt (4 g) is obtained with high purity. HPLC purity = 99.8%; assay = 94.5% (KBr) 3269, 1747, 1668 cm⁻¹.

### Example 2

Crude Cefdinir (10 g) is suspended in water (170 ml) and ethyl acetate (12 ml), then a diluted ammonium hydroxide solution is added drop by drop, adjusting pH to 6.5 and keeping the temperature at 5°C, until a solution is obtained. Ammonium dihydrogen phosphate (5.8 g) and seed crystals of Cefdinir ammonium salt are added and the solution is slowly stirred at 5°C for one hour, adjusting pH to 6.5 by addition of a diluted ammonium hydroxide solution. The crystallized product is filtered and washed first with water then with isopropanol. After drying crystalline Cefdinir ammonium salt (6.5 g) identical to the product of example 1 is obtained with high purity.

### Example 3

Crude Cefdinir (10 g) is suspended in water (170 ml) and ethyl acetate (12 ml), then a diluted ammonium hydroxide solution is added drop by drop, adjusting pH to 6.5 and keeping the temperature at 5°C, until a solution is obtained. Ammonium dihydrogen phosphate (11.6 g) and seed crystals of Cefdinir ammonium salt are added and the solution is slowly stirred at 5°C for one hour, keeping the pH at 6.5 by addition of a diluted ammonium hydroxide solution. The crystallized product is filtered and washed first with water and then with isopropyl alcohol. After drying, crystalline Cefdinir ammonium salt (8 g) identical to the product of example 1 is obtained with high purity.

### Example 4

Crude Cefdinir (10 g) is suspended in water (170 ml) and ethyl acetate (12 ml), then a diluted ammonium hydroxide solution is added drop by drop, adjusting pH to 6.5 and keeping the temperature at 5°C, until a solution is obtained. Ammonium hydrogen phosphate (17.4 g) and seed crystals of Cefdinir ammonium salt are added and the mixture is slowly stirred at 5°C for one hour, adjusting pH at 6.5 by addition of a diluted ammonium hydroxide solution. The crystallized product is filtered and washed first with water then with isopropanol. After drying, crystalline Cefdinir ammonium salt (9.4 g) identical to the product of example 1 is obtained with high purity.

### Example 5

Cefdinir phosphate (10 g) is suspended in water (112.5 ml) and ethyl acetate (7.5 ml), then a diluted ammonium hydroxide solution is added drop by drop, adjusting the pH to 6.5 and keeping the temperature at 5°C, until a solution is obtained. Ammonium dihydrogen phosphate (7.5 g) and seed crystals of Cefdinir ammonium salt are added and the mixture is slowly stirred at 5°C for one hour, adjusting pH at 6.5 by addition of a diluted ammonium hydroxide solution. The crystallized product is filtered and washed first with water then with isopropanol. After drying, crystalline Cefdinir ammonium salt (6.2 g) identical to the product of example 1 is obtained with high purity.

### Example 6

Cefdinir phosphate (10 g) is suspended in water (112.5 ml) and ethyl acetate (7.5 ml), then a diluted ammonium hydroxide solution is added drop by drop, adjusting the pH to 6.5 and keeping the temperature at 5°C, until a solution is obtained. Sodium dihydrogen phosphate (14 g) and seed crystals of Cefdinir ammonium salt are added and the solution is slowly stirred at 5°C for one hour, keeping pH at 6.5 by addition of a diluted solution of ammonium hydroxide. The crystallized product is filtered and washed first with water then with isopropanol. After drying crystalline Cefdinir ammonium salt (5.9 g) identical to the product of example 1 is obtained with high purity.

### Example 7

Cefdinir phosphate (10 g) is suspended in water (112.5 ml) and ethyl acetate (7.5 ml), then a diluted ammonium hydroxide solution is added drop by drop, adjusting the pH to 6.5 and keeping the temperature at 5°C, until a solution is obtained. Sodium monohydrogen phosphate (14.4 g) and seed crystals of Cefdinir ammonium salt are added and the solution is slowly stirred at 5°C for one hour. The crystallized product is filtered and washed first with water then with isopropanol. After drying crystalline Cefdinir ammonium salt (5.5 g) identical to the product of example 1 is obtained with high purity.

### Example 8

Crude Cefdinir (10 g) is suspended in water (170 ml) and ethyl acetate (12 ml), then a diluted ammonium hydroxide solution is added drop by drop, adjusting pH to 6.5 and keeping the temperature at 5°C, until a solution is obtained. Ammonium chloride (20 g) and seed crystals of Cefdinir ammonium salt are added and the solution is slowly stirred at 5°C for one hour adjusting pH at 6.5 by addition of a diluted ammonium hydroxide solution. The crystallized product is filtered and washed first with water and then with isopropanol. After drying, crystalline Cefdinir ammonium salt (8.9 g) identical to the product of example 1 is obtained with high purity.

### Example 9

Crude Cefdinir (10 g) is suspended in water (170 ml) and ethyl acetate (12 ml), then a diluted ammonium hydroxide solution is added drop by drop, adjusting pH to 6.5 and keeping the temperature at 5°C, until a solution is obtained. Sodium chloride (30 g) and seed crystals of Cefdinir ammonium salt are added and the solution is slowly stirred at 5°C for one hour. The crystallized product is filtered and washed first with water then with isopropanol. After drying, crystalline Cefdinir salt ammonium (9.1 g) identical to the product described in example 1 is obtained with high purity.

### Preparation of Cefdinir

### Example 10

Cefdinir ammonium salt (10 g) is dissolved in water saturated with ethyl acetate (630 ml) at a temperature of 5°C and the solution is treated with active charcoal. The pH of the clarified solution is adjusted to 2.5 with diluted hydrochloric acid. The crystallized product is filtered and washed in sequence with water then with isopropanol. After drying, crystalline Cefdinir monohydrate (8.7 g) is obtained with high purity.

Water (K.F.) = 5.5%. IR: (KBr) 3300, 1786, 1752, 1667, 1610, 1544 cm⁻¹.

### Example 11

Cefdinir ammonium salt (10 g) is dissolved in water saturated with ethyl acetate (330 ml) at a temperature of 5°C and the solution is treated with active charcoal. The temperature of the clarified solution is set to 35°C and the pH is adjusted to 2.2 by addition of diluted hydrochloric acid. The crystallized product is filtered and washed with water. After drying, Cefdinir crystalline form A (8.2 g) is obtained with high purity. The resulting product is crystalline and shows an IR spectrum (KBr: 1765, 1685, 1543 cm⁻¹) and X ray diffractogram identical to those reported in example 4 of US 4,935,507.

## Claims

1. Crystalline Cefdinir ammonium salt of formula (I) 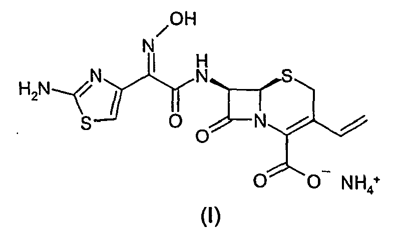 **characterized by** the following diffraction spectrum:
| Angle | d value | Intensity |
|---|---|---|
| (2-Theta) | (Angstrom) | (%) |
| 10.592 | 8.34491 | 39.7 |
| 12.091 | 7.31366 | 56.1 |
| 16.726 | 5.29604 | 41.2 |
| 18.023 | 4.91778 | 39.0 |
| 19.191 | 4.62106 | 96.5 |
| 19.850 | 4.46905 | 30.6 |
| 21.396 | 4.14949 | 100.0 |
| 22.876 | 3.88425 | 78.1 |
| 25.150 | 3.53798 | 49.9 |
| 25.603 | 3.47638 | 65.8 |
| 26.150 | 3.40491 | 42.2 |
| 26.845 | 3.31826 | 37.0 |
| 29.699 | 3.00563 | 37.0 |
| 30.121 | 2.96449 | 33.7 |
| 33.560 | 2.66810 | 41.0 |
| 34.658 | 2.58607 | 25.1 |
| 36.262 | 2.47524 | 18.3 |
| 36.841 | 2.43766 | 17.7 |
| 37.426 | 2.40094 | 17.6 |
| 38.220 | 2.35287 | 22.4 |
| 39.155 | 2.29881 | 16.9 |
| 40.016 | 2.25128 | 15.9 |
| 41.219 | 2.18834 | 15.6 |
| 41.779 | 2.16027 | 17.3 |
| 42.610 | 2.12004 | 16.8 |
| 46.508 | 1.95102 | 12.9 |
| 50.510 | 1.80542 | 11.9 |
| 51.487 | 1.77343 | 11.3 |
| 52.638 | 1.73733 | 13.3 |

2. Use of the salt of claim 1 for the preparation of Cefdinir.

## Patentansprüche

1. Kristallines Cefdinir Ammoniumsalz der Formel (I) 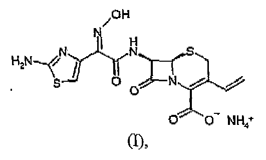 das durch das folgende Beugungsspektrum charakterisiert ist:
| Winkel | d Wert | Intensität |
|---|---|---|
| (2-Theta) | (Angström) | (%) |
| 10.592 | 8.34491 | 39.7 |
| 12.091 | 7.31366 | 56.1 |
| 16.726 | 5.29604 | 41.2 |
| 18.023 | 4.91778 | 39.0 |
| 19.191 | 4.62106 | 96.5 |
| 19.850 | 4.46905 | 30.6 |
| 21.396 | 4.14949 | 100.0 |
| 22.876 | 3.88425 | 78.1 |
| 25.150 | 3.53798 | 49,9 |
| 2:5.603 | 3.47638 | 65.8 |
| 26.150 | 3.40491 | 42.2 |
| 26.845 | 3.31826 | 37.0 |
| 29.699 | 3.00563 | 37.0 |
| 30.121 | 2.96449 | 33.7 |
| 33.560 | 2.66610 | 41.0 |
| 34.658 | 2.58607 | 25.1 |
| 36.262 | 2.47524 | 18.3 |
| 36.841 | 2.43766 | 17.7 |
| 37.426 | 2.40094 | 17.6 |
| 38.220 | 2.35287 | 22.4 |
| 139.155 | 2.29881 | 16.9 |
| 40.0115 | 2.25128 | 15.9 |
| 41.219 | 2.18834 | 15.6 |
| 41.779 | 2.16027 | 17.3 |
| 42.610 | 2.12004 | 16.8 |
| 46.508 | 1.95102 | 12.9 |
| 50.510 | 1.80542 | 11.9 |
| 51.487 | 1.77343 | 11.3 |
| 52.638 | 1.73733 | 13.3 |

2. Verwendung des Salzes gemäß Anspruch 1 zur Herstellung von Cefdinir.

## Revendications

1. Sel d'ammonium cristallin de Cefdinir de formule (I) 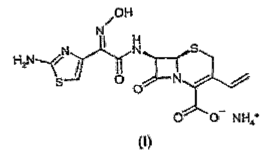 **caractérisé par** le spectre de diffraction suivant :
| Angle | Valeur d | Intensité |
|---|---|---|
| (2-thêta) | (angström) | (%) |
| 10,592 | 8,34491 | 39,7 |
| 12,091 | 7,31366 | 56,1 |
| 16,726 | 5,29604 | 41,2 |
| 18,023 | 4,91778 | 39,0 |
| 19,191 | 4,62106 | 96,5 |
| 19,850 | 4,46905 | 30,6 |
| 21,396 | 4,14949 | 100,0 |
| 22,876 | 3,88425 | 78,1 |
| 25,150 | 3,53798 | 49,9 |
| 25,603 | 3,47638 | 65,8 |
| 26,150 | 3,40491 | 42,2 |
| 26,845 | 3,31826 | 37,0 |
| 29,699 | 3,00563 | 37,0 |
| 30,121 | 2,96449 | 33,7 |
| 33,560 | 2,66810 | 41,0 |
| 34,658 | 2,58607 | 25,1 |
| 36,262 | 2,47524 | 18,3 |
| 36,841 | 2,43766 | 17,7 |
| 37,426 | 2,40094 | 17,6 |
| 38,220 | 2,35287 | 22,4 |
| 39,155 | 2,29881 | 16,9 |
| 40,016 | 2,25128 | 15,9 |
| 41,219 | 2,18834 | 15,6 |
| 41,779 | 2,16027 | 17,3 |
| 42,610 | 2,12004 | 16,8 |
| 46,508 | 1,95102 | 12,9 |
| 50,510 | 1,80542 | 11,9 |
| 51,487 | 1,77343 | 11,3 |
| 52,638 | 1,73733 | 13,3 |

2. Utilisation du sel selon la revendication 1 pour la préparation du Cefdinir.
